# EUROPÄISCHE PATENTSCHRIFT

(11) **EP 1 508 308 B1**
(45) Veröffentlichungstag und Bekanntmachung des Hinweises auf die Patenterteilung: **19.12.2007**
(21) Anmeldenummer: 04014428.9
(22) Anmeldetag: 19.06.2004
(51) Int. Cl.: A61B 17/92, A61B 17/72, A61B 17/17

(54) **Kombination aus intramedulärem Nagel und Ziel- und/oder Einschlaginstrument**
Combination of an intramedullary pin and a targering device or/and a percussion device
Combinaison d'un clou intramedullaire et d'un instrument de centrage ou/et de percussion

(30) Priorität: 30.07.2003 DE 20311718 U
(43) Veröffentlichungstag der Anmeldung: 23.02.2005
(73) Patentinhaber: Stryker Trauma GmbH, 24232 Schönkirchen (DE)
(72) Erfinder: Völzow, Stefan, 24248 Mönckeberg (DE)
(74) Vertreter: Kopf, Korbinian Paul

(56) Entgegenhaltungen:
- EP-A- 0 091 499
- EP-A- 0 888 751
- DE-A- 3 003 957

## Beschreibung

Die Erfindung bezieht sich auf eine Kombination aus intramedulärem Nagel und Ziel- und/oder Einschlaginstrument nach dem Obergriff des Anspruchs 1.

Es ist bekannt, intrameduläre Knochennägel mit einem Einschlaginstrument zu koppeln, um sie in den Knochenkanal vorzutreiben. Bei Verriegelungsnägeln, die mit Querbohrungen versehen sind für die Hindurchführung von Verriegelungsschrauben, ist auch bekannt, das Einschlaginstrument gleichzeitig als Zielgerät auszuführen. Ein Zielgerät dient dazu, die Querbohrungen, die beim Verriegelungsnagel im Knochen nicht sichtbar sind, aufzufinden. Zu diesem Zweck weist das Zielgerät, das zugleich als Einschlaginstrument dient, neben einem Anschlußende zur Verbindung mit dem einen Ende des Nagels einen Zielarm auf, der sich annähernd parallel und im Abstand zum Nagel erstreckt. Damit die Querbohrungen im Nagel aufgefunden werden können, ist erforderlich, daß der Nagel relativ zum Ziel- und Einschlaginstrument eine vorgegebene Drehlage aufweist. Es ist daher bekannt, den Nagel endseitig mit einer oder mehreren achsparallelen Ausnehmungen zu versehen und das Ziel- und Einschlaginstrument mit einem oder mehreren achsparallelen Vorsprüngen. Greifen Vorsprung und Ausnehmung annähernd formschlüssig ineinander, ist die vorschriftsmäßige Drehlage erreicht. Es ist ferner bekannt, mit Hilfe einer Schraube Zielgerät und Nagel axial fest miteinander zu koppeln. Zu diesem Zweck weist der hohle Endabschnitt des Nagels einen Gewindeabschnitt auf und das Anschlußende des Zielgerätes ist mit einer axialen Schraube versehen, die in das Innengewinde des Nagels eingeschraubt wird, um die Teile axial fest aneinander zu spannen.

Bevor Nagel und Zielgerät miteinander verbunden, d. h. verschraubt werden, ist bei den herkömmlichen Systemen erforderlich, den Nagel in vorschriftsmäßiger Ausrichtung gegen das Anschlußende des Zielgeräts zu halten. Hierfür sind bereits beide Hände des Anwenders in Anspruch genommen. Es ist daher relativ aufwendig bzw. kompliziert, außerdem die beschriebene Schraubverbindung zu bewerkstelligen.

Der Erfindung liegt daher die Aufgabe zu Grunde, eine Kombination aus intramedulärem Nagel und Ziel- und/oder Einschlaginstrument so auszubilden, daß die Verbindung dieser beiden Teile von einem Anwender ohne weiteres herbeigeführt werden kann.

Diese Aufgabe wird durch Merkmale des Anspruchs 1 gelöst.

Bei der erfindungsgemäßen Kombination weist das Anschlußende des Zielund/oder Einschlaginstruments einen Endabschnitt auf, der annähernd passend in einen endseitigen Bohrungsabschnitt des Nagels eingreift. Endabschnitt und Bohrungsabschnitt weisen Mittel auf für eine lösbare Schnappverbindung der Teile bei gleichzeitigem Eingriff von achsparallelem Vorsprung und achsparalleler Ausnehmung.

Die erfindungsgemäße Kombination hat den Vorteil, daß die zweite Hand des Anwenders, die üblicherweise den Nagel in Position halten muß, frei ist, um die beiden Teile miteinander zu verschrauben.

Die Schnappverbindung zwischen Nagel und Anschlußende des Ziel- und/oder Einschlaginstruments hat lediglich die Aufgabe, die Teile vorübergehend im vorschriftsmäßiger Zuordnung zueinander zu halten, bis über die Schraubverbindung eine endgültige Festlegung erfolgt. Daher braucht die Schnappverbindung nur so ausgeführt zu sein, daß relativ geringe Kräfte erforderlich sind, um Nagel und Anschlußende des Ziel- und/oder Einschlaginstruments miteinander zu verrasten. Dies erleichtert auch das Lösen der Teile voneinander, wenn der Nagel endgültig eingetrieben und die Schraube aus dem Nagel entfernt ist.

Nach einer Ausgestaltung der Erfindung weist der Endabschnitt des Anschlußendes des Ziel- und/oder Einschlaginstruments an seinem Umfang einen radial nachgebenden Abschnitt auf, der beim Einsetzen des Endabschnitts in den Bohrungsabschnitt des Nagels radial nach innen verformt wird und mit einer Rastschulter oder dergleichen im Bohrungsabschnitt zusammenwirkt. Das Anschlußende des Instruments kann daher einteilig mit dem Endabschnitt ausgeführt sein, wobei lediglich Schwächungen oder Schlitze dafür sorgen, daß ein radial nachgebender Abschnitt gebildet ist. Es versteht sich, daß dieser relativ zum Durchmesser des Bohrungsabschnitts im Nagel ein geringes Übermaß hat, damit der radial nachgebende Abschnitt eine Schulter oder Ausnehmung hintergreifen kann, wenn die Teile zusammengesteckt werden. Die Schulter kann zum Beispiel von einer ringförmigen Nut im Bohrungsabschnitt des Nagels gebildet sein.

Bei einer alternativen Ausführungsform weist der Endabschnitt des Ziel- und/oder Einschlaginstruments eine ringförmige Nut auf, in welcher eine ringförmige Feder so aufgenommen ist, daß sie radial nach innen verformbar ist und im entspannten Zustand radial etwas über den Umfang des Endabschnitts übersteht. Der endseitige Bohrungsabschnitt weist eine ringförmige Nut auf, in welche sich die Feder radial aufweitet, wenn der Endabschnitt in den Bohrungsabschnitt eingesteckt wird. Die Feder ist zum Beispiel eine geschlitzte Ringfeder.

Die ringförmige Nut im Endabschnitt des Ziel- und/oder Einschlaginstruments kann nach einer weiteren Ausgestaltung der Erfindung durch einen Steg unterbrochen sein. Dieser verhindert eine unerwünschte Drehung der ringförmigen Feder in der Nut.

Um die Eintreibkräfte wirksam auf den Nagel zu übertragen, sieht eine Ausgestaltung der Erfindung vor, daß das Anschlußende des Ziel- und/oder Einschlaginstruments eine radiale Anschlagfläche aufweist, die gegen das freie Ende des Nagels anliegt, wenn die lösbare Schnappverbindung hergestellt ist.

Die Erfindung wird nachfolgend anhand eines in Zeichnungen dargestellten Ausführungsbeispiels näher erläutert.
- Fig. 1: zeigt perspektivisch ein Abschlußende eines Ziel- und/oder Einschlaginstruments nach der Erfindung.
- Fig. 2: zeigt einen Schnitt durch die Darstellung nach Fig. 3 entlang der Linie 2-2.
- Fig. 3: zeigt eine Seitenansicht des Anschlußendes nach Fig. 1.
- Fig. 4: zeigt vergrößert den Teil 4 nach der Darstellung von Fig. 3.
- Fig.: 5 zeigt einen Schnitt durch einen Femurnagel zur Versorgung von trochanteren oder Schenkelhalsfrakturen.
- Fig. 6: zeigt vergrößert den Teil 6 des Nagels nach Fig. 5.
- Fig. 7: zeigt vergrößert den Teil 7 der Darstellung nach Fig. 6.

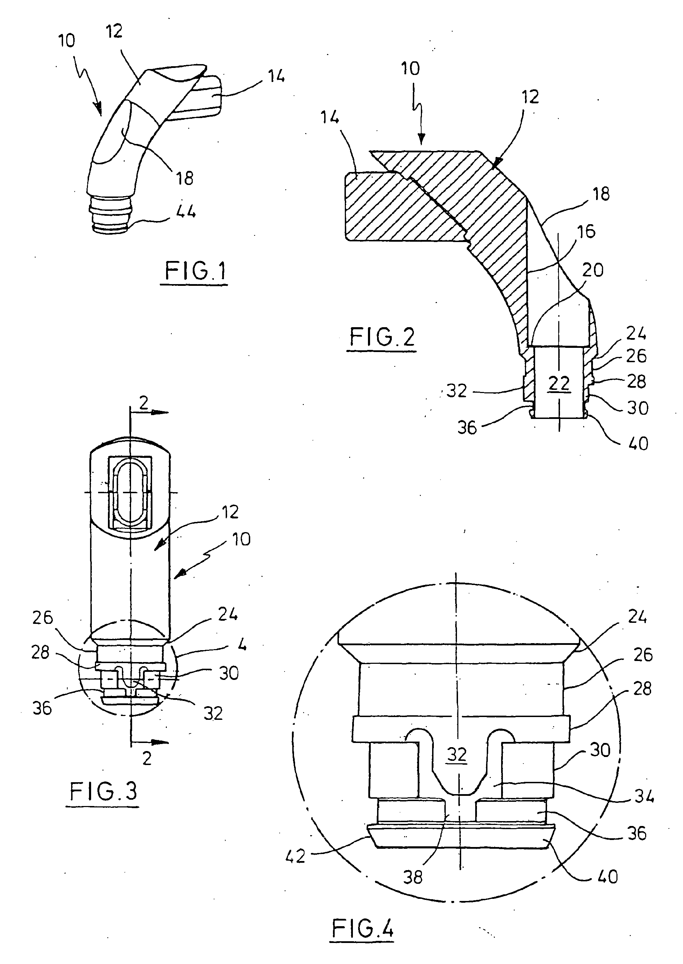

In Fig. 1 ist ein Anschlußende 10 eines nicht weiter dargestellten Ziel- und/oder Einschlaginstruments dargestellt, das aus einem eigentlichen Anschlußteil 12 und einem Teil 14 besteht, welches letzteres dazu dient, eine Verbindung mit dem übrigen Teil des Ziel- und Einschlaginstruments herzustellen, was jedoch im einzelnen nicht gezeigt und auch bekannt ist.

Wie aus Fig. 2 zu erkennen, weist das Anschlußteil 12 eine gerade Durchbohrung 16 auf, die am oberen Ende in einer schrägen ovalen Öffnung 18 endet. Innerhalb der Bohrung 16 ist eine Ringschulter 20 gebildet. Unterhalb der Ringschulter 20 ist ein Bohrungsabschnitt 22 von konstantem Durchmesser vorgesehen: An der Außenseite des im Querschnitt kreisförmigen Abschnitts des Anschlußteils unterhalb der Ringschulter 22 befindet sich eine konische Stufe 24 und unterhalb dieser ein erster zylindrischer Abschnitt 26, an den sich ein zweiter zylindrischer Abschnitt 28 von etwas größerem Durchmesser anschließt. Unterhalb des zylindrischen Abschnitts 28 ist ein weiterer zylindrischer Abschnitt 30 von geringerem Durchmesser vorgesehen, der aber nur teilzylindrisch ist, weil er auf der Seite des Teils 14 durch einen axial sich erstreckenden radialen Vorsprung 32 unterbrochen ist. Dieser Vorsprung 32 ist in Fig. 4 gut zu erkennen. Er hat einen Außendurchmesser wie der zylindrische Abschnitt 28 und steht somit radial über den Abschnitt 30 über, welcher bei 34 zu beiden Seiten des Vorsprungs 32 abgeflacht ist, damit der Vorsprung 32 radial ausreichend erhaben ist.

Unterhalb des zylindrischen Abschnitts 30 ist eine Ringnut 36 geformt, welche durch einen achsparallelen Steg 38 unterbrochen ist. Unterhalb der Ringnut 36 ist ein konischer Abschnitt 40 vorgesehen, dessen Außenfläche 42 sich nach unten verjüngt. Die Ringnut 36 dient zur Aufnahme einer geschlitzten Ringfeder, wobei die Enden der Feder auf beiden Seiten des Steges 38 liegen (nicht gezeigt) mit einem geringen Abstand von diesem. Eine solche Feder ist bei 44 in Fig. 1 angedeutet.

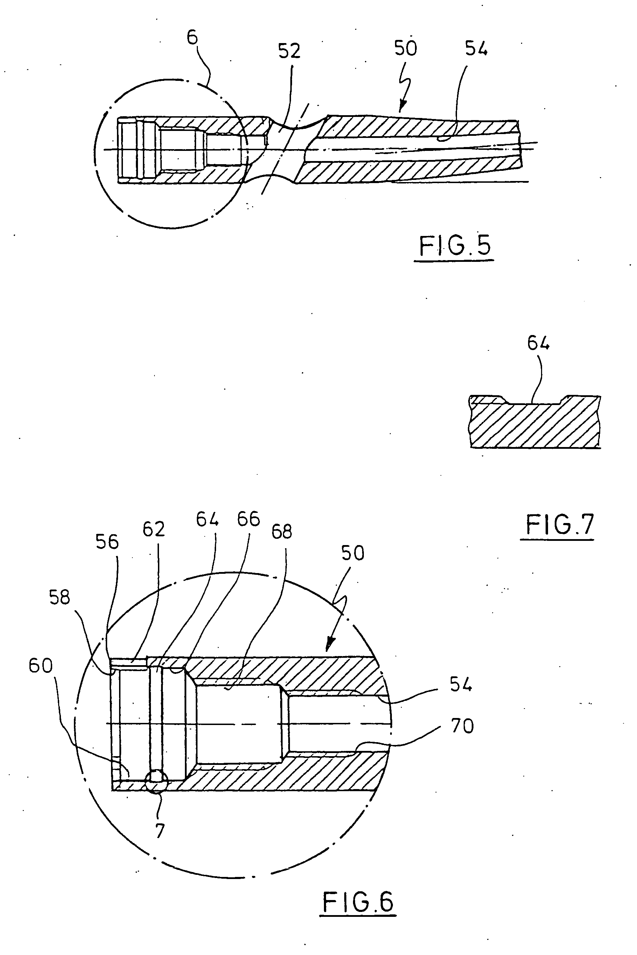

In Fig. 5 ist ein proximaler Endabschnitt 50 eines nicht weiter dargestellten Femurnagels im Schnitt gezeigt, der eine schräge Durchbohrung 52 aufweist, für die Auf nahme einer nicht gezeigten Schenkelhalsschraube. Der Nagel weist eine axiale Durchbohrung 54 auf. Die weiteren Teile des Endabschnitts 50 werden anhand von Fig. 6 erläutert.

Der Nagel hat eine freie Stirnfläche 56, an welche sich ein konischer Einführabschnitt 58 anschließt. An diesen schließt sich ein zylindrischer Bohrungsabschnitt 60 an, wobei die Wandung des Nagels im Bereich des Bohrungsabschnitts 60 und des Einführabschnitts 58 eine achsparallele Ausnehmung 62 aufweist. An den Bohrungsabschnitt 60 schließt sich eine ringförmige Nut 64 an und an diese ein zylindrischer Bohrungsabschnitt 66 mit etwas kleinerem Durchmesser als die Nut 64. In Fig. 7 ist die Nut 64 deutlicher herausgestellt. Man erkennt, daß die Wände der Nut 64 schräg sind.

Weiter vom Ende des Nagels entfernt liegt ein erster Innengewindeabschnitt 68 und diesem folgend ein zweiter Gewindeabschnitt 70 von geringerem Durchmesser.

Der größte Außendurchmesser des Abschnitts 40 (Fig. 4) entspricht annähernd dem Innendurchmesser des Bohrungsabschnitts 60, welcher gleich ist dem Innendurchmesser des Bohrungsabschnitts 66. Der in Fig. 2 bis 4 dargestellte Endabschnitt des Anschlußteils 12 kann daher in die Bohrungsabschnitte 60 und 66 eingeführt werden, wenn der achsparallele nasenartige Vorsprung 32 zur Ausnehmung 62 ausgerichtet ist. Diese beiden Abschnitte greifen annähernd passend ineinander. Der konische Einführabschnitt 58 und der konische Abschnitt 40 des Abschlußteils erleichtern das Zusammenstecken. Das Einstecken des Endabschnitts des Anschlußteils 12 ist beendet, wenn die dem Nagel zugekehrte Schulterfläche des zylindrischen Abschnitts 28 gegen die Endfläche 56 des Nagels anschlägt. Ist dies der Fall, dann liegen Nut 36 am Anschlußteil 12 und Nut 64 vom Nagel 50 einander gegenüber. Befindet sich nun in der Nut 36 die bereits erwähnte Ringfeder 44, die gegenüber dem Durchmesser des Bohrungsabschnitts 60 ein gewisses radiales Übermaß hat, schnappt die Ringfeder 48 in die Nut 64 und legt dadurch die beiden Teile axial zueinander fest. Der Nagel 50 ist daher in Drehrichtung und axial in richtiger Lage mit dem Anschlußende 10 des Ziel- und/oder Einschlaginstruments verbunden. Nunmehr kann durch eine Schraube oder eine Schraubhülse eine feste Verbindung dieser beiden Teile vorgenommen werden. Nach dem Eintreiben des Nagels in den Knochenkanal und Lösen der Schraubverbindung kann das Instrument durch einen geringen Kraftaufwand wieder vom Nagel gelöst werden. Es versteht sich, daß als Schraube, welche durch die Bohrung 16 (Fig. 2) hindurchgeführt wird und mit dem Gewindeabschnitt 68 des Nagels zusammenwirkt, eine Hülse verwendet werden kann, wie sie etwa in US 5,176,681 beschrieben ist. Die Schraube wirkt mit dem Gewindeabschnitt 68 des Nagels zusammen. Mit dem Gewindeabschnitt 70 des Nagels 50 kann ein Verriegelungsstift (nicht gezeigt) zusammenwirken, um eine (nicht gezeigte) Schenkelhalsschraube, die durch die Querbohrung 52 geführt ist, in gewünschter Weise festzulegen.

## Patentansprüche

1. Kombination aus intramedulärem Nagel und Ziel- und/oder Einschlaginstrument, wobei ein Anschlußende des Ziel- und/oder Einschlaginstruments einen achsparallelen Vorsprung und ein Anschlußende des Nagels eine achsparallele Ausnehmung aufweisen, um Ziel- und/oder Einschlaginstrument und Nagel in ihrer Drehlage zueinander festzulegen, wenn sie axial aneinander stoßen und wobei ferner Verbindungsmittel vorgesehen sind, um Ziel- und/oder Einschlaginstrument und Nagel axial fest, aber lösbar miteinander zu verbinden, **dadurch gekennzeichnet, daß** das Anschlußende des Ziel- und/oder Einschlaginstruments einen Endabschnitt (30, 40) aufweist, der annähernd passend in einen endseitigen Bohrungsabschnitt (60, 66) des Nagels (50) eingreift und Endabschnitt und Bohrungsabschnitt Mittel aufweisen für eine lösbare Schnappverbindung der Teile bei gleichzeitigem Eingriff von achsparallelem Vorsprung (32) und achsparalleler Ausnehmung (62).

2. Kombination nach Anspruch 1, **dadurch gekennzeichnet, daß** der Endabschnitt des Anschlußendes des Ziel- und/oder Einschlaginstruments am Umfang einen radialen nachgebenden Abschnitt aufweist, der beim Einsetzen des Endabschnitts in den Bohrungsabschnitt radial nach innen verformt wird und mit einer Rastschulter oder dergleichen im Bohrungsabschnitt zusammenwirkt.

3. Kombination nach Anspruch 2, **dadurch gekennzeichnet, daß** der Bohrungsabschnitt eine Ringnut (64) aufweist, in die der radial nachgebende Abschnitt des Anschlußendes des Ziel- und/oder Einschlaginstruments eingreift.

4. Kombination nach Anspruch 1, **dadurch gekennzeichnet, daß** der Endabschnitt des Ziel- und/oder Einschlaginstruments eine ringförmige Nut (36) aufweist, in welcher eine Ringfeder (44) aufgenommen ist derart, daß sie radial nach innen verformbar ist und im entspannten Zustand radial etwas über den Umfang des Endabschnitts übersteht und der endseitige Bohrungsabschnitt des Nagels (50) eine ringförmige Nut (64) aufweist, in welche sich die Feder radial aufweitet, wenn der Endabschnitt in den Bohrungsabschnitt eingesteckt wird.

5. Kombination nach Anspruch 4, **dadurch gekennzeichnet, daß** eine geschlitzte Ringfeder vorgesehen ist.

6. Kombination nach Anspruch 4, **dadurch gekennzeichnet, daß** die ringförmige Nut (36) im Endabschnitt des Ziel- und/oder Einschlaginstruments durch einen Steg (38) unterbrochen ist.

7. Kombination nach einem der Ansprüche 1 bis 6, **dadurch gekennzeichnet, daß** das Anschlußende (10) des Ziel- und/oder Einschlaginstruments eine radiale Anschlagfläche aufweist, gegen welche das freie Ende (56) des Nagels (50) anliegt, wenn die lösbare Schnappverbindung hergestellt ist.

## Claims

1. Combination of an intramedullary nail and a targeting and/or striking instrument, wherein one connecting end of the targeting and/or striking instrument has an axially parallel protrusion and one connecting end of the nail has an axially parallel recess in order to fix the targeting and/or striking instrument and nail in their rotational position in relation to each other when they abut each other axially and wherein connecting means are also provided in order to connect the targeting and/or striking instrument and nail in an axially fixed but detachable manner to each other, **characterised in that** the connecting end of the targeting and/or striking instrument comprises an end section (30, 40) which engages in an approximate fit in a terminal borehole section (60, 66) of the nail (50) and the end section and borehole section have means for a detachable snap connection of the parts with the simultaneous engagement of the axially parallel protrusion (32) and axially parallel recess (62).

2. Combination according to claim 1, **characterised in that** the end section of the connecting end of the targeting and/or instrument comprises on its perimeter a radial yielding section which is deformed radially inward when the end section is inserted in the borehole section and interacts with a detent shoulder or the like in the borehole section.

3. Combination according to claim 2, **characterised in that** the borehole section comprises an annular groove (64) into which the radially yielding section of the connecting end of the targeting and/or striking instruments engages.

4. Combination according to claim 1, **characterised in that** the end section of the targeting and/or striking instrument comprises a ring-shaped groove (36) in which an annular spring (44) is accommodated in such a way that it can be deformed radially inwardly and in relaxed state protrudes radially slightly over the perimeter of the end section and the terminal borehole section of the nail (50) comprises a ring-shaped groove (64), in which the spring expands radially when the end section is inserted into the borehole section.

5. Combination according to claim 4, **characterised in that** a slotted annular spring is provided.

6. Combination according to claim 4, **characterised in that** the ring-shaped groove (36) in the end section of the targeting and/or striking instrument is interrupted by a web (38).

7. Combination according to any one of claims 1 to 6, **characterised in that** the connecting end (10) of the targeting and/or striking instrument comprises a radial stop surface against which the free end (56) of the nail (50) lies when the detachable snap connection is established.

## Revendications

1. Combinaison d'un clou intramédullaire et d'un instrument de centrage et/ou de percussion, dans laquelle une extrémité de raccordement de l'instrument de centrage et/ou de percussion comporte une saillie d'axe parallèle et une extrémité de raccordement du clou comporte un évidement d'axe parallèle, pour immobiliser fermement l'instrument de centrage et/ou de percussion et le clou l'un par rapport à l'autre dans leur position de rotation lorsqu'ils s'assemblent axialement et dans laquelle des moyens de liaison sont par ailleurs prévus pour relier axialement l'un à l'autre l'instrument de centrage et/ou de percussion et le clou de manière ferme mais amovible, **caractérisée en ce que** l'extrémité de raccordement de l'instrument de centrage et/ou de percussion comporte une section d'extrémité (30, 40) qui vient en prise de manière approximativement ajustée dans une section de perçage (60, 66) du côté extrémité du clou (50), et la section d'extrémité et la section de perçage comportent des moyens permettant une liaison par encliquetage amovible des pièces avec engrènement simultané de la saillie d'axe parallèle (32) et de l'évidement d'axe parallèle (62).

2. Combinaison selon la revendication 1, **caractérisée en ce que** la section d'extrémité de l'extrémité de raccordement de l'instrument de centrage et/ou de percussion comporte sur le pourtour une section radialement élastique qui, lors de la mise en place de la section d'extrémité dans la section de perçage, se déforme radialement vers l'intérieur et coopère avec un épaulement d'encliquetage ou équivalent dans la section de perçage.

3. Combinaison selon la revendication 2, **caractérisée en ce que** la section de perçage comporte une gorge annulaire (64), dans laquelle vient en prise la section radialement élastique de l'extrémité de raccordement de l'instrument de centrage et/ou de percussion.

4. Combinaison selon la revendication 1, **caractérisée en ce que** la section d'extrémité de l'instrument de centrage et/ou de percussion comporte une gorge annulaire (36) dans laquelle un ressort annulaire (44) est logé de telle façon qu'il est déformable radialement vers l'intérieur et, à l'état détendu, dépasse radialement légèrement au-dessus du pourtour de la section d'extrémité et la section de perçage du côté extrémité du clou (50) comporte une gorge annulaire (64), dans laquelle le ressort s'élargit radialement, lorsque la section d'extrémité est insérée dans la section de perçage.

5. Combinaison selon la revendication 4, **caractérisée en ce qu'**il est prévu un ressort annulaire fendu.

6. Combinaison selon la revendication 4, **caractérisée en ce que** la gorge annulaire (36) dans la section d'extrémité de l'instrument de centrage et/ou de percussion est interrompue par une nervure (38).

7. Combinaison selon l'une des revendications 1 à 6, **caractérisée en ce que** l'extrémité de raccordement (10) de l'instrument de centrage et/ou de percussion comporte une surface de butée radiale contre laquelle l'extrémité libre (56) du clou (50) repose, lorsque la liaison par encliquetage amovible est établie.
